# EUROPEAN PATENT SPECIFICATION

(11) **EP 1 147 218 B1**
(45) Date of publication and mention of the grant of the patent: **16.03.2005**
(21) Application number: 00900727.9
(22) Date of filing: 21.01.2000
(51) Int. Cl.: C12Q 1/68, C12N 15/10

(54) **SELECTION PROCEDURE USING PRODRUG/ENZYME SYSTEM**
SELEKTIONSVERFAHREN MIT WIRKSTOFFSVORSTUFE-ENZYM SYSTEM
PROCEDE DE SELECTION PAR SYSTEME DE PROMEDICAMENTS/ENZYMES

(30) Priority: 22.01.1999 GB 9901471; 22.01.1999 US 116924 P
(43) Date of publication of application: 24.10.2001
(73) Proprietor: ML Laboratories PLC, London W1S 1HU (GB)
(72) Inventor: SEARLE, Peter F., University of Birmingham, Birmingham B15 2TT (GB)
(74) Representative: Goddard, David John
(86) International application number: PCT/GB2000/000157
(87) International publication number: WO 2000/043541

(56) References cited:
- WO-A-91/19818
- HUBER B E ET AL: "VDEPT: AN ENZYME/PRODRUG GENE THERAPY APPROACH FOR THE TREATMENT OFMETASTATIC COLORECTAL CANCER" ADVANCED DRUG DELIVERY REVIEWS,NL,AMSTERDAM, vol. 17, no. 3, 5 December 1995 (1995-12-05), pages 279-292, XP000654839 ISSN: 0169-409X
- FRIEDLOS F ET AL: "GENE-DIRECTED ENZYME PRODRUG THERAPY: QUANTITATIVE BYSTANDER CYTOTOXICITY AND DNA DAMAGE INDUCED BY CB1954 IN CELLS EXPRESSING BACTERIAL NITROREDUCTASE" GENE THERAPY,GB,MACMILLAN PRESS LTD., BASINGSTOKE, vol. 5, no. 1, January 1998 (1998-01), pages 105-112, XP000783674 ISSN: 0969-7128

## Description

The present invention relates to a process for the selection from a gene library of a gene encoding an enzyme capable of catalysing the conversion of a prodrug to its active drug form. Prodrug activating enzymes convert prodrugs, which are relatively non-toxic, to cytotoxic agents that have been shown to be effective in killing tumour cells (Connors, T.A., (1995) Gene Therapy 2:702-709). It has been hypothesised that selective delivery of prodrug activating enzymes to tumours would allow the local generation of a cytotoxic agent, through the systemic administration of a substrate prodrug. This would allow a greater differential between systemic toxicity and anti-tumour activity than can be achieved by systemic delivery of cytotoxic agents.

The activating enzyme may be targeted to the tumour cells by coupling it to a tumour-targeting moiety such as an antibody of suitable specificity (''antibody-directed enzyme prodrug therapy", = ADEPT). Alternatively, a gene encoding the prodrug activating enzyme may be delivered to the tumour cells, either as some kind of DNA complex, or in various kinds of viral vectors (GDEPT or VDEPT, = gene / virus directed enzyme prodrug therapy). (Huber *et al.,* 1991; Huber *et al.,* 1994; Harris *et al.,* 1994; Searle et *al.,* 1996).

Similar systems may be effective in other instances requiring selective cell killing, such as, for example, countering restenosis of blood vessels following balloon angioplasty, selective tissue ablation in transgenic animals and as a fail-safe suicide system to allow killing of (genetically modified) immune-effector cells present in allogeneic bone-marrow transplants.

One known prodrug activating enzyme is nitroreductase from *E*. *coli,* which can activate the prodrug CB1954 (5-[aziridin-1-yl]-2,4-dinitrobenzamide) by reduction at either the 2 or the 4 position. Further, non-enzymatic reaction of this product leads to the generation of a potent bifunctional alkylating agent which can produce interstrand crosslinks in DNA, and which is therefore highly cytotoxic. Experiments using this approach have demonstrated that retroviral transfer of a nitroreductase expression cassette can sensitise a number of colorectal, pancreatic and ovarian tumour cell lines to the prodrug CB1954 (McNeish *et al.,* 1998: Green *et al.*, 1996). Significantly increased survival and apparent cures have also been observed following treatment of mice harbouring nitroreductase-expressing tumours with CB1954 (McNeish *et al.,* 1998).

However, the efficiency of this method of selective tumour cell killing appears presently to be sub-optimal, partly due to the difficulty of achieving sufficiently high levels of gene transfer to a sufficient number of tumour cells *in vivo.* The ultimate therapeutic potential of the system in treating cancer patients will depend upon a number of factors. including the efficiency with which the gene encoding the prodrug activating enzyme can be delivered to and expressed within the tumour cells to produce the enzyme and the selectivity of these processes for tumour cells versus the cells of normal, vital tissues or organs within the patient. Clinical efficacy will also depend upon a number of factors such as the intrinsic susceptibility of the tumour cells to the activated prodrug, and the capacity of host defence mechanisms such as the immune system to work co-operatively with the gene therapy to assist in killing tumour cells that are not directly killed by the activated prodrug.

A number of other parameters also appear to be important. For example, the concentration of prodrug to which the cells are exposed is a major factor determining the rate at which the activated drug form can be generated to produce its toxic effect. Furthermore. the greater the duration of exposure of the cell population to the prodrug, the greater the amount of activated drug form which can be generated. In the body, the substrates for the enzymatic reaction are likely to be present in non-limiting amounts, (although this will of course depend upon the dose which has been administered, and the pharmacokinetics of its distribution within the body) so the rate at which the activated drug species can be generated, at any given local concentration of prodrug, will depend upon the amount of catalytically-active enzyme that is available in the cell. Perhaps most important is the efficiency of the enzyme; at a given concentration of substrate and enzyme, the reaction will proceed faster if the K_{M} is lower for substrate, or if the k_{cat} is greater, or both.

Naturally, one would attempt to optimise the exposure (concentration x duration) of the tumour to the prodrug by selecting the most suitable route and schedule of delivery, however it is likely that the maximum achievable exposure would be limited by factors such as toxicity to the patient or solubility of the prodrug. However, there are in principle two routes to "improve" the enzyme. One involves site-directed mutagenesis. An alternative route requires no prior knowledge of the structure of the enzyme, but relies on the production of a large number of random mutants, and the use of one or more screening procedures to identify the rare, improved variants against the large background of indifferent or detrimental mutants.

Such strategies of random mutagenesis and selection have been shown to yield improved enzyme variants. For example, Black *et al.* introduced random sequence into a region of the thymidine kinase gene from herpes simplex virus. and screened more than one million independent clones by positive selection on plates containing 5-fluorodeoxyuridine, to identify 426 which retained a threshold level of thymidine kinase activity. These were then screened individually to identify a subset that demonstrated improved characteristics with the prodrugs acyclovir and/or ganciclovir (Black *et al.,* 1996). Another group applied the PCR-shuffling technique with alternating rounds of selection and PCR shuffling has resulted in a 32,000-fold enhancement of the activity of TEM-1 β-lactamase against the antibiotic cefotaxime (Stemmer, 1994); selection was accomplished by plating bacteria containing the plasmid library on agar containing the antibiotic.

However, although such precedents provide encouragement that it is possible to make improved enzymes via such mutagenesis strategies, no efficient selection strategies have yet been described which could be generally applied to the improvement of any prodrug activating enzyme. Consequently, there remains a need for a system that allows the selection of variant prodrug activating enzymes that exhibit improved catalytic activity.

### Summary of the invention

According to the present invention there is provided a method of selection of a nucleic acid encoding an enzyme that is capable of converting a prodrug to its active drug form comprising the steps of: contacting a population of bacteria transformed with a bacteriophage library with a prodrug in a medium, wherein;
i) the transformed bacteria are in the lysogenic state, and
ii) when converted to its active drug form, the prodrug causes activation of the proteolytic activity of bacterial RecA and lysis of the bacteria;
separating bacteriophage particles released by lysis of the bacteria from said medium, and analysing the genotype of said separated bacteriophage for a nucleic acid encoding the enzyme.

By prodrug is meant any drug molecule that is itself relatively inert pharmaceutically but which has a pharmacological effect once activated in an organism by biological, biochemical, chemical or physical means. The enzyme encoded by the selected nucleic acid should be a prodrug activating enzyme that acts catalytically to convert prodrug to its active drug form. Alternatively, there may be more than one reaction step between the prodrug and the final chemical species which is directly toxic; for example, the toxic 5-[aziridin-1-yl]-4-hydroxyiamino-2-nitrobenzamide which can be produced by nitroreductase from CB1954 is believed to undergo further, non-enzymatic reaction to generate 4-(N-acetoxy)-5-[aziridin-1-yl]-2-nitrobenzamide, which is believed to be the chemical species which reacts with DNA to produce the toxic crosslinks (Knox, R. J., F. Friedlos, T. Marchbank, and J. J. Roberts. ( 1991), *Biochem. Pharmacol.* 42:1691-1697). The toxic product cannot be generated in any significant quantity, without the presence of the activating enzyme.

The method of the invention requires the use of a temperate bacteriophage and is based on the ability of such bacteriophage to propagate in bacteria in either of two modes. The first mode (the lytic phase) involves rapid, episomal replication of the bacteriophage leading to assembly of progeny virus particles and lysis of the infected bacteria cell. The other mode (the lysogenic phase) does not kill the host bacterium: instead, the bacteriophage genome becomes covalently integrated into the DNA of the bacterial chromosome, where it is replicated along with the host DNA, and is transmitted to the daughter cells. Once established, the lysogenic state is maintained by the *c*I repressor protein of the bacteriophage, which binds to sequences within the bacteriophage genome to promote its own synthesis, and repress transcription of the remainder of the bacteriophage genome. In this state, the bacterium is protected from further infection by other, similar bacteriophages, because the presence of the *c*I repressor in the cell also prevents the expression of genes required for lytic growth from incoming bacteriophage genomes. In this state, the bacterium is called a "lysogen'' and the integrated copy of the bacteriophage is called a prophage. Establishment of this state is called lysogeny (Hendrix, R. W., J. W. Roberts, F. W. Stahl, and R. A. Weisberg (1983) Lambda II).

Certain treatments of lysogenic bacteria can lead to activation of a proteolytic activity of the bacterial RecA protein. This in turn can lead to cleavage and hence inactivation of the bacteriophage *c*I repressor protein, thus triggering lytic growth of the prophage. The inactivation of *c*I repressor allows expression of the bacteriophage genes required for lytic growth, including functions which excise the prophage genome from the bacterial chromosome, leading to its episomal replication, and the assembly of many copies of the bacteriophage into mature bacteriophage particles, which are then released upon lysis of the host cell. (Roberts, J. W., and R. Devoret. *(1983)* Lysogenic induction, in R. W. Hendrix, J. W. Roberts, F. W. Stahl and R. A. Weisberg (ed.), Lambda 11; Cold Spring Harbor Laboratory Press).

In the temperate bacteriophage Lambda, prophage induction can be induced in this manner by agents that damage DNA (for example, ultraviolet light, ionising radiation, activated chemical carcinogens. certain anti-tumour drugs), as well as agents which disrupt DNA replication. including 5-fluorouracil, and nucleoside- or nucleotide-analogues. The mechanism is thought to be that any of these agents lead to the generation of single-stranded DNA in the cell, which is required, along with ATP or dATP and magnesium ions, to activate a specific protease activity of bacterial RecA. This normally leads, via cleavage of the LexA protein (a repressor of transcription), to the activation of a number of genes in the bacterium associated with DNA repair functions, allowing better survival of the DNA damage; this is called the SOS response.

The principle of the invention is that a library of DNA molecules encoding a number of variants of prodrug activating enzymes is inserted into the genome of a bacteriophage. The bacteriophage vectors are then introduced into host bacteria, and lysogens are selected. In general, each bacterium contains only one bacteriophage vector type and the inserted gene is expressed within the lysogenic bacteria. Upon exposure of the population of lysogenic bacteria to prodrug, the toxic derivative of the prodrug will only be produced within bacterial cells containing a prodrug activating enzyme, and then at a rate which depends upon the catalytic activity of the prodrug activating enzyme that is present within that cell. The presence of the toxic product within the cell leads to the activation of the protease activity of the bacterial RecA protein, leading to cleavage of the cI repressor protein of the bacteriophage, and thus triggering lytic growth. The prophage in that particular cell is thus excised and replicated, leading to lysis of the host cell and the release of around 100 progeny bacteriophage particles into the medium, from which they can be readily recovered.

At any given concentration of prodrug, the toxic product will be produced faster in bacteria containing a bacteriophage vector encoding a more efficient enzyme. Lytic growth of bacteriophage will therefore be triggered relatively early in those instances when the bacteriophage encodes a highly active prodrug activating enzyme. Lytic growth will be triggered relatively late (or not at all above the background level), if the encoded enzyme variant has little or no activity against the prodrug. Thus. at limiting exposure to prodrug (low concentration and/or short duration of exposure). the copies of the vector recovered are enriched in those encoding the most efficient variants of the prodrug activating enzyme.

Any temperate bacteriophage may be used in the invention, as will be clear to those of skill in the art. The bacteriophage lambda (λ) is the bacteriophage of choice. All genes and functions of the temperate bacteriophage that are required for the initial infection, and establishment and maintenance of lysogeny of bacteria should be retained. Furthermore, all genes and functions required for the induction and completion of the lytic cycle upon activation of RecA protease activity should be present in the bacteriophage.

As will be clear to the skilled artisan, other features of the bacteriophage are desirable. For example, the bacteriophage genome should contain a restriction site at which a DNA fragment (encoding a prodrug activating enzyme) can be inserted and expressed in the lysogenic state. The presence of suitably positioned regulatory DNA sequences to facilitate the expression of the inserted DNA in the bacterial lysogen is also advantageous, since it removes the need for the inserted DNA to contain its own regulatory sequences required for expression of the encoded proteins. The insertion site could be designed so as to increase the efficiency of insertion of suitable DNA fragments, for example, by the use of suitable, asymmetrical sites to favour the insertion of the exogenous DNA in a preferred orientation, and to reduce the background level of non-productive ligation products. This would facilitate the generation of a library having a high level of diversity. The inclusion of a selectable marker such as an antibiotic-resistance gene within the bacteriophage vector would facilitate the separation of lysogens from bacteria not infected with the bacteriophage vector. The bacteriophage could also be designed to express its encoded protein only in the lysogenic state. As will be clear to the skilled artisan, this might be by using a regulated promoter allowing selective expression, for example, using the tac promoter and the inducer IPTG in the media.

Methods for the introduction of genes or gene fragments into bacteriophage DNA and for the construction of bacteriophage libraries are known in the art, for example, see Sambrook *et al.,* 1989, Cold Spring Harbor Laboratory Press. Of course, it is not necessary for the entire gene for the prodrug activating enzyme to be cloned into the bacteriophage DNA - fragments may also be used. However, if a fragment is used, this should encode a protein fragment that retains catalytic activity in activating prodrug to its active drug form.

The bacteriophage library should contain a library of genes. A library of genes may be used that is derived from a single organism. to select for a gene or genes which exhibit the desired catalytic activity, for example as a form of expression cloning to identify novel active proteins in a particular organism. However, in most instances the gene encoding the wild type enzyme of interest will already be available in the art - nucleic acid coding sequences for use in the invention are widely reported in the scientific literature and are also available in public databases. Further, the DNA may be commercially available, may be part of known cDNA libraries or may be generated using standard molecular biology and/or chemistry procedures as will be clear to those of skill in the art. In such instances. the invention will be most useful to allow the selection of genes encoding improved variants of the wild type protein that exhibit improved catalytic activity. Accordingly, a gene library will preferably comprise a selection of mutated variants of the wild type gene.

Many different approaches to random mutagenesis may be used to generate the library. For example, chemical modification can be used to alter bases in the gene sequence, leading to changes in the DNA sequence upon replication or repair. Examples of chemical modification include treatment of a single stranded form of the gene with bisulphite, which converts some of the cytosine residues to uracil residues. When the opposite strand of DNA is then made by a DNA polymerase, an adenine residue is inserted opposite the uracil nucleotide. In this way, a C-G base-pair is converted to a U-A base-pair, and, subsequently, to T-A (see Watson, J. D., M. Gilman, J. Witkowski, and M. Zoller, (1992) Recombinant DNA. 2^{nd} Edition; Scientific American Books; W. H. Freeman & Co. New York, and references therein). DNA polymerases with relatively high error rates can be used for replication of the gene e.g. the avian myeloblastosis reverse transcriptase can be used in this way (Singh, M., S. Heaphy, and M. J. Gait. ( 1986), Prot. Eng. **1**:75-76; see also Abarzua, P and Marians, K J 1984; *Proc. Natl. Acad. Sci. USA* **81**, 2030-2034), as can the *Thermus aquaticus* (Taq) DNA polymerase, which may be used repeatedly as in the polymerase chain reaction (Leung, D W, Chen, E and Goeddel, D V (1989); *Technique* **1**; 11 - 15). Another mutagenesis strategy has been described by Stemmer (Stemmer, 1994); in this method, the gene is randomly fragmented and then reassembled by a process similar to the polymerase chain reaction, with an associated introduction of mutations. An advantage of this particular strategy lies in the combination of alternating rounds of selection, and the mutagenic "PCR-shuffling" procedure, which can recombine beneficial mutations from different gene variants into a single gene, and facilitate the separation of deleterious changes from those that are beneficial.

An approach intermediate between that of site-directed mutagenesis and completely random mutagenesis throughout the gene might be appropriate where some information has indicated that particular regions of the enzyme are likely to be critically involved in the reaction of interest, but it has not yet proven feasible to predict precisely how the enzyme may be improved. In such a situation, it may be appropriate to produce a library of mutants in which the changes are localised to that critical region of the protein. This could be achieved using recombinant DNA techniques to replace the relevant region of the gene with a diversity of nucleic acid molecules that are completely random in sequence (with or without variation in length) to make a library of gene sequences. Clearly, the majority of mutations generated by randomised approaches are unlikely to result in improvement of the enzyme, and many changes are likely to be detrimental. However, the method of the invention allows the selection of beneficial variants out of a large negative background, meaning that the proportion of detrimental mutation events is largely irrelevant.

A further aspect of the invention may involve a PCR-shuffling and selection process with a family of related genes, rather than using a single gene. For example, in the case of nitroreductase, genes from various different bacteria (e.g. species of *Salmonella, Enterobacter, Helicobacter* or *Vibrio)* might be used in the selection process. This procedure would facilitate sampling of a larger volume of ''sequence-space" giving, greater diversity in the library, and hence more efficient generation of improved derivatives (Crameri *et al.,* 1998).

It is envisaged that there could be synergy between the "random" approach, and the structure-guided, site-directed mutagenesis approach. Structural analysis of improved variants would help evaluate how the enzyme works, and structure-guided, site-directed mutants could be optimised by random shuffling mutagenesis and selection.

The bacteriophage library will generally comprise around 10⁶ variants, as is typical with lambda libraries, although either smaller or larger libraries could be used. After introduction of the gene library into bacteriophage, the bacteriophage library is propagated in bacteria and may be stored for subsequent use (see Sambrook *et al.,* 1989).

Choice of host bacteria is also of importance. The host bacterium must support the lytic and lysogenic growth of bacteriophage vector and must retain the ability of the wild-type RecA protein to activate lysogens into lytic growth upon exposure to agents which damage DNA or otherwise disrupt DNA synthesis in the bacterium. Many bacterial strains are suitable for the method of the invention. although *E. coli* is preferred. The production of lysogens will be facilitated if the bacterium has a mutation such as *hflA*^{*-*} (high frequency of lysogeny), which favours the establishment of lysogeny. Without this host mutation, most bacteriophage would first grow lytically, and lysogenised bacteria would only emerge against a background of lysis. The presence of an antibiotic-resistance gene in the bacteriophage is thus useful to assist the selection of lysogenised bacteria. as those bacteria which were not infected and thus cannot become lysogenised will be inhibited or killed by the antibiotic. Other forms of selection are also possible,for example, based on complementation of metabolic/nutritional deficiencies of an appropriate host strain.

In general, each lysogenised bacterium will contain only a single bacteriophage genome, since the initial infection will be performed with an excess of bacteria relative to infectious bacteriophage particles; and once lysogeny is established, the cI repressor in the cell prevents expression of lytic cycle genes and of the genes required for integration, from the genomes of infecting bacteriophages.

A further advantageous feature of the bacterial host strain, to facilitate the selection procedure, is that the bacteria should be negative for the prodrug activating enzyme. For example, many strains of *E. coli* contain an endogenous, functional nitroreductase gene. A suitable deletant strain may be publicly or commercially available. If not, the generation of a strain in which the enzyme is deleted will be within the normal skills of those practised in the art. Preferably, the strain used should lack a functional nitroreductase.

The bacterial strain *E. coli* C600*Hfl* (Promega cat. No. D3131) contains a functional nitroreductase gene. This gene could be mutated, and deletants selected for. Other methods of mutation will be clear to the skilled artisan, for example, by first introducing a mutation into a cloned copy of the nitroreductase gene, such as by: insertion of the omega-(Ω)-interposon described by Prentki, P and Krisch, H.M., [1984], Gene **29**;303-313 or *in vitro* insertional mutagenesis with a selectable DNA fragment in which a streptomycin/spectinomycin-resistance gene is flanked by translational and transcriptional termination signals. The mutated gene could then be introduced into the bacteria (without any attached replication origin), and the bacteria selected with streptomycin or spectinomycin; thus one would select for bacteria in which the mutated gene had recombined into the host genome, and these could then be checked to confirm the inactivation of the endogenous nitroreductase gene.

In the method of the invention, the bacteriophage library must be introduced into host bacteria and the lysogenic state established. Under growth conditions the bacteria multiply, replicating their own DNA and that of the bacteriophage, so that copies of the lysogenic bacteriophage are transmitted to daughter cells.

Under conditions of environmental stress, and in particular in response to DNA damage, the SOS pathway is activated in the bacterium, activating machinery required for repair of the DNA damage. The inducing signal is also a signal for reactivation of lysogenic bacteriophage. Specifically, the RecA protease of the bacterium cleaves the *c*I repressor of the bacteriophage, allowing lytic cycle genes to be expressed. The bacteriophage genome is excised from the bacterial genome, replicated, and assembled into progeny bacteriophage particles, which are released by lysis of the host cell. Drugs that act on DNA, causing single-stranded lesions, crosslinking or breakage of the DNA strands, or drugs which act in other ways to inhibit the process of DNA replication, can all lead to the generation of an excess of single-stranded DNA in the cell and hence activate this pathway. Accordingly, those cells containing active prodrug activating enzyme will convert more prodrug to its active drug form and will consequently lyse earlier than cells containing a less efficient enzyme.

There will be a background of spontaneous bacteriophage release, which will contaminate the "selected'' bacteriophage population. As will be clear to the skilled artisan, the relative numbers of background and selected bacteriophage can be managed by appropriate choice of prodrug concentration and duration of exposure (which will determine the fraction of the culture in which lysis is induced).

Preferably, the bacteriophage selected can be used immediately to generate more lysogens for further iterative rounds of selection; each round of selection will reduce the fraction of irrelevant bacteriophage in the "background". The selected variant genes could then be isolated,for example, by appropriate restriction endonuclease digestion or by PCR and used for further rounds of shuffling (to allow recombination of beneficial mutations from different bacteriophages) and mutagenesis, then inserted back into the bacteriophage vector and the selection process repeated. In this fashion, progressive improvement of the selected gene populations results. Preferably, at least two. more preferably three or more rounds of shuffling followed by selection are used.

After each round of the selection, some of the variant genes may be analysed directly (for example, by first plaqueing the phage to isolate single clones, then sequencing; by prodrug activation-mediated cytotoxicity in bacteria, or by enzymological analysis after expression in bacteria). The most promising of the variants can be used directly for antibody-directed enzyme prodrug therapy (ADEPT) or (after optional modification of codon usage) for gene/virus directed enzyme prodrug therapy (G/VDEPT) applications. It is also possible to "backcross" the optimised constructs with the original nitroreductase gene during a round of shuffling and selection. to identify which amino acid substitutions are truly beneficial and which are "passenger mutations'' of little consequence for the functionality of the enzyme.

The method of the invention is suitable for any combination of activating enzyme and prodrug, provided that prodrug activation results in activation of the proteolytic activity of the bacterial RecA protein. Nitroreductase is one example of such an enzyme and CB 1954 is an example of a suitable prodrug substrate. CB1954 is a relatively poor substrate for wild type nitroreductase: the turnover rate k_{cat} is just 360 min⁻¹ and the Michaelis constant K_{M} is high at 860 µM (9). This enzyme is thus an ideal choice for the selection method of the present invention. Other enzymes which might be suitable for optimisation for their prodrugs include thymidine kinase, cytosine deaminase or purine nucleoside phosphorylase. The mammalian DT-diaphorase enzymes, which can also activate CB 1954 though less efficiently than nitroreductase, could also be optimised for this purpose. Other examples will be clear to those of skill in the art.

The choice of prodrug will depend on the system being studied, and will clearly be a substrate for the prodrug activating enzyme of interest. In the case of thymidine kinase, the prodrugs acyclovir and/or ganciclovir will be suitable. For cytosine deaminase, 5-fluorocytosine is converted to the active form 5-fluorouracil. For purine nucleoside phosphorylase, the product of the *E. coli DeoD* gene, 6-methyl-9-(2-deoxy-β-D-erythro-pentofuranosyl) purine (6-MPDR, a deoxyadenosine analogue) is an example of a suitable prodrug. Prodrug substrates other than CB1954 have been described for nitroreductase (Anlezark *et al.,* 1995; Friedlos *et al.,* 1997). Some of these prodrugs have certain advantages over CB1954, for example, SN 23862 (the bischloroethyl analogue of CB 1954) is not activated by mammalian DT diaphorases, and so may have lower toxicity. 2-[N,N-bis(2-iodoethyl)amino]-3,5-dinitrobenzamide, which has shown promising properties *in vitro,* could be applied to the method of the present invention. Similarly, other enzymes could be tested with the same prodrugs, for example, rat DT diaphorase has the advantage of selectively reducing CB 1954 at the 4 position, although the k_{cat} is rather lower than for nitroreductase. Nitroreductases from bacteria other than *E*. *coli* provide alternative starting points. Thus, one could in principle start with several different nitroreductase genes from different families to facilitate sampling of a larger volume of "sequence-space" (Crameri *et al.,* 1998).

The concentration of prodrug to be used in the method of the invention is selected so as to facilitate the selection process and will thus depend on factors such as the relative proportion of the library which is thought to harbour active enzyme, the size of the library and the approximate activity of the optimal enzyme. For example, at low concentrations of prodrug, only lysogens containing the most efficient enzymes should within a reasonable time convert adequate prodrug to generate DNA damage sufficient to activate RecA protease activity and hence induce lytic growth of the bacteriophage in that bacterium. Thus, at limiting concentration of prodrug, only the bacteriophage containing the "best" prodrug activating enzyme genes are activated into the lytic pathway. This concentration of prodrug thus allows recovery of variant genes that made their bacterial hosts most susceptible to prodrug.

In the case of nitroreductase and CB 1954, a suitable concentration of prodrug is around 10 - 100µM. For acyclovir, gancyclovir, 5-fluorocytosine etc, a suitable concentration is around 0.1 - 1 µg/ml.

Suitable media in which to carry out the method of the present invention will be clear to those of skill in the art. For example, for *E.coli*,Luiria-Bertani (LB) broth is suitable. In addition to prodrug, the media may contain other necessary additives, for example, antibiotic or an inducer, for example, IPTG.

Bacteriophage particles released by lysis of host bacteria may easily be separated from non-lysed bacteria. For example, treatment of media samples with chloroform (~1 -10%) is sufficient to kill any bacteria. The lysate can then be centrifuged briefly and the supernatant will contain released bacteriophage that can be used to propagate in bacteria and to select further for bacteriophage containing genes of interest.

According to a further aspect of the invention there is provided a catalytic enzyme or fragment thereof, isolated according to a method described above. Preferably, the catalytic enzyme will comprise an improved variant of wild type nitroreductase, thymidine, kinase, cytosine deaminase or purine nucleotide phosphorylase. The amino acid sequence of the protein of protein fragment will differ from the wild type sequence of the protein fragment, by amino acid insertion, deletion or substitution.

Also described is a nucleic acid molecule encoding a catalytic enzyme selected according to the method of the invention. Such a nucleic acid molecule may be obtained by sequence analysis of the genome of bacteriophage selected for containing an enzyme variant of improved catalytic activity in the particular system studied. It is envisaged that information obtained by such sequencing will be useful to allow analysis of the structure of the protein of interest and will allow the further improvement of the protein activity by rational or random mutagenic strategies.

The invention will now be described by way of example, with particular emphasis on the enzyme nitroreductase and prodrug CB 1954. It will be appreciated that modification of detail may be made without departing from the scope of the invention.

### BRIEF DESCRIPTION OF THE FIGURES

Figure 1 is a graph showing the induction of phage release from 5:1 mixture of lysogens JG3J1 (control) and JG16C1(ntr) by 0.5mM CB 1954.

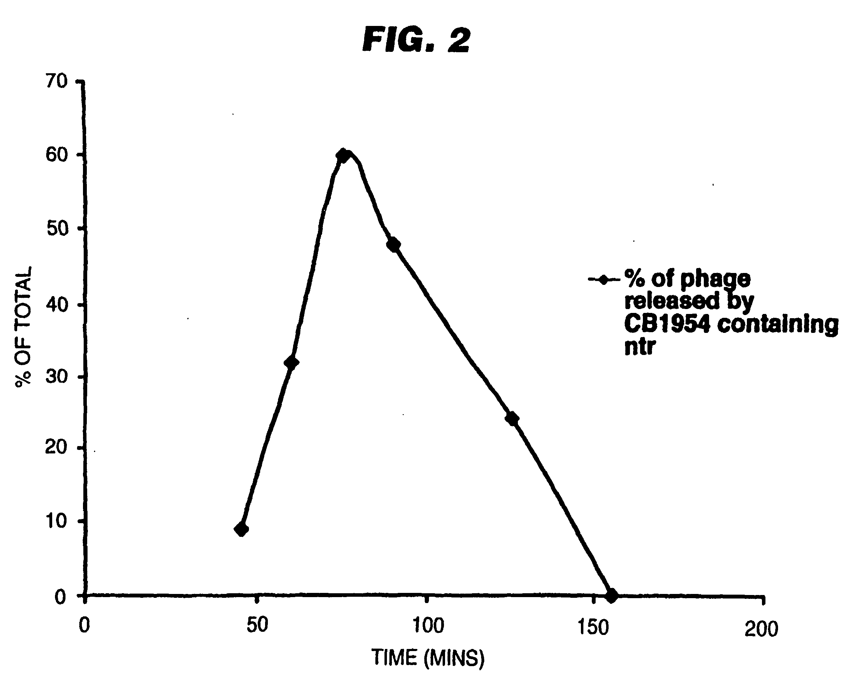

Figure 2 shows the type of phage released from lysogen mixture, as determined by PCR.
Figure 3 shows the amount of nitroreductase when fully induced in λJG3J1 lysogens.

### EXAMPLES

### 1. Construction of a suitable vector

Information relating to recombinant DNA technology techniques can be found in (Sambrook *et al.,* 1989). In addition, information of the biology of the bacteriophage lambda can be found in (Hendrix. *et al.,* 1983).

### Preparation of a promoter cassette.

A DNA fragment containing the tac promoter was obtained from plasmid pDR540 (Pharmacia), by PCR amplification using primers 5'- ACCTGACGTCTAAGAAAC -3' and 5'- GCTCTAGATTGTTATCCGCTCAC -3'. The amplified DNA fragment was cleaved with restriction endonucleases *Eco*RI and *Xba*I, and the major fragment (369 bp) was cloned between the *Eco*RI and *Xba*I sites of the widely used vector plasmid, pUC19, to generate plasmid pPS1133C2. The sequence of the insert was verified by DNA sequencing (Sanger & Coulson, 1977).

The tac promoter is a composite promoter, made from parts of the trp promoter and the lac promoter; it allows a high level of expression, which can be repressed by the lac repressor; thus, a high level of expression can be induced in the presence of inducers such as IPTG.

### Insertion of paired SfiI sites, to facilitate directional cloning of suitable inserts.

The two oligonucleotides PS1133A (5'- CTAGGGCCTGCGAGGCCTTAATTAA-GGCCTCCCGGGCCT -3') and PS1133B (5'- CTAGAGGCCCGGGAGGCCTTAA-TTAAGGCCTCGCAGGCC -3') were annealed together to generate a short piece of DNA containing two *Sfi*l sites separated by a *Pac*I site, and with 4 nucleotide 5' extensions on either end compatible with ligation into *Xba*I sites. However, only that at the right end regenerates the *Xba*I site:

SfiI                    PacI   SfiI

PS1133A5'CTAGGGCCTGCGAGGCCTTAATTAAGGCCTCCCGGGCCT     3'

PS1133B 3'    CCGGACGCTCCGGAATTAATTCCGGAGGGCCCGGAGATC 5'

This was ligated into the *Xba*I site of plasmid pPS1133C2. A derived plasmid clone named pPS1133L30 was identified, in which a single copy of the 39bp insert was present within the plasmid, in the orientation which regenerated the *Xba*I site downstream of the insert (i.e. the tac promoter, and the *Xba*I site are on opposite sides of the inserted 39 bp sequence).

### Production of a plasmid with paired SfiI sites

The plasmid pET11c (Novagen) was cleaved with *Xba*I, and the following annealed oligonucleotides were cloned into that site:

PS1134A 5 CTAGAGGCCTGCGAGGC 3

PS1134B 3     TCCGGACGCTCCGGATC 5

The resultant plasmid pPS1134D4 was identified with the single insert in the intended orientation, which regenerated the *Xba*I site on the upstream side of the insert (i.e. nearer to the promoter).

Plasmid pPS1134D4 was cleaved with *Bam*HI, and the following annealed oligonucleotides were cloned into that site:

PS1134C 5' GATCCGGCCTCCCGGCC 3'

PS1134D 3'     GCCGGAGGGCCCGGCTAG 5'

The resultant plasmid pPS1134S5 has a single copy of the insert in the orientation which regenerates the *Bam*HI site upstream (towards the promoter).

Thus, the coding region of genes can be inserted between an *Nde*I (at the initiation codon) and a *Bam*HI site in this vector. The vector then provides a "Shine-Dalgarno" sequence an appropriate distance upstream of the start codon, to facilitate binding of ribosomes to the RNA transcribed from the gene, and hence efficient translation to produce the encoded protein. This is flanked on either side by the paired *Sfi*I sites, allowing intervening fragment to be cloned directionally into the bacteriophage vector to be described. The T7 promoter present upstream of this cassette in pPS1134S5 also allows efficient expression of the protein from this plasmid, in a suitable host strain such as *E*. *coli* BL21 [λDE3].

### Insertion of the E. coli nitroreductase gene into pPS1134S5

DNA sequence derived from the nitroreductase gene *(nfnB)* of E. *coli* strain DH5α was amplified by the polymerase chain reaction from genomic DNA purified from that strain, using primers PS1138A 5'- GGGAATTCCATATGGATATCATTTCTGTCGCCTTAAAGC -3' and

PS1138B 5'- CGCGGATCCTGAGAGGAAATAGCCGGGCAGATGC -3'.

The amplified DNA was digested with the enzymes *Bam*HI and *Nde*I, and the main fragment (~700 bp) was cloned between the *Nde*I and *Bam*HI sites of pPS1134S5, to produce pJG16C1; the sequence of the inserted fragment containing the nitroreductase coding sequence was verified by DNA sequencing.

### Insertion of the tac promoter cassette into lambda λNM1151

The lambda vector λNM1151 (Murray, 1983) appeared to meet most of the essential criteria, although the immunity region of this bacteriophage is derived from bacteriophage 21, and is reported as imm²¹*c*I^{ts}, i.e. a temperature-sensitive mutant of the repressor. The bacteriophage 21 is closely related to lambda, however it was not clear whether the induction from lysogeny mediated by the imm²¹*c*Its would be as efficient as that with the canonical, imm^{λ}*c*I⁺. This λNM1151 vector had unique restriction enzyme sites for *Eco*RI, *Hind*III and *Bam*HI. It was therefore decided to produce the vector based on this bacteriophage, however it would be possible. if the imm²¹*c*I^{ts} proved less than ideal, to carry out a genetic cross with wild-type λ to swap the imm²¹*c*I^{ts} for imm^{λ}*c*I⁺.

A sample of λNM115 bacteriophage was provided by Prof. N. Murray (University of Edinburgh). The tac promoter cassette, including the pair of *Sfi*I sites, was obtained as a *Hind*III fragment from pPS1133L10, and cloned into the *Hind*III site of λNM1151 to generate λPS1141A5.

### Insertion of a kanamycin-resistance cassette into λPS1141A5.

A *Nhe*I-*Bam*HI fragment encoding the aminoglycoside 3'-phosphotransferase gene (kanamycin resistance) was isolated from pACYC177 (New England Biolabs). The ends were made blunt by treatment with *Pfu* DNA polymerase (Stratagene), and the fragment was cloned into pHP45Ω which had been digested with *Sma*I, allowing the kanamycin resistance gene to replace the "interposon" in that vector (Prentki, P., and H. M. Krisch. 1984) . This step allowed the kanamycin resistance gene to be subsequently isolated as an *Eco*RI fragment, which was inserted into the single *Eco*RI site of λPS1141A5, to generate λJG3J1.

### Insertion of the E. coli nitroreductase gene into the expression site of λJG3J1.

The nitroreductase gene of *E*. *coli* strain DH5α was excised from plasmid pJG16C1 by digestion with *Sfi*I, and inserted between the two *Sfi*I sites of λJG3J1. The asymmetry of the *Sfi*I sites ensured that the nitroreductase gene could only be inserted in the orientation required to allow its expression from the tac promoter. The resulting bacteriophage was called λJG16C1.

### 2. Recovery of catalytic bacteriophage

### AIM:

To test whether the vector λG16C1 (encoding a functional, wild-type nitroreductase) could be preferentially recovered from a mixture with λJG3J1 (lacking a nitroreductase insert), by treatment of the mixed population of lysogens with CB1954.

### Method:

*E. coli* C600*Hfl* (obtained from Promega Cat No. D3131) were infected with λJG3J1 or λJG16C1 for 15min at 37°C and spread onto kanamycin (30µg/ml) selective Lennox agar (LA) plates to select for lysogens. A single colony of each was picked. and the lysogenic bacteria were then grown aerobically at 37°C to mid log phase in LB containing 0.1mM IPTG. Approximately 4.8 x 10⁸ λJG3J1 lysogens and 9 x 10⁷ λJG16C1 lysogens (as estimated from the optical density; approximate 5:1 ratio without:with nitroreductase) were mixed in 5ml LB containing 0.1mM IPTG in the presence or absence of 500 µM CB1954 (provided by Cobra Therapeutics and diluted from a 50mM stock solution in 2:7 N-methyl pyrrolidinone:PEG300). The mixtures of bacterial lysogens were incubated at 37°C with shaking and 50µl samples removed at intervals between 15 and 185 minutes after mixing.

Each sample was immediately mixed with 1ml LB and 100µl chloroform to kill any bacteria, briefly centrifuged to pellet debris, and the supernatant diluted I in 10 before using 10 µl to infect *E. coli* C600*Hfl* and plating onto LA containing kanamycin, to select for bacteria lysogenised by bacteriophage in the sample.

The number of colonies growing from the samples was counted the following day, and indicates the total number of bacteriophage present in the sample.

The polymerase chain reaction (PCR) was used to determine whether the bacteria in each colony contained λJG3J1 or λJG16C1. A number of individual colonies were picked at random from the most relevant plates and transferred to a 204µl PCR tube, lysed in a microwave for 2min and 35µl of PCR reaction mix [16mM (NH₄)₂SO₄, 67mM Tris-Cl (pH 8.8 at 25°C), 0.01% Tween-20 (Bioline), 0.2mM each dNTP, 1.5mM MgCl₂, 1u Biotaq (Bioline)] plus 0.25 µM primers JG2A 5'-TGGCGGAAAGGTATGCATGC[?] and; JG2B 5'-CAGAGCATTAGCGCAAGGTG, which anneal to λ sequences flanking the *Hind*III site, was added.

A tube containing reaction mix but no bacteria was used as a control to check for contamination of PCR reaction components. After 30 cycles (94°C/5min; then 30x 94°C/45s, 55°C/45s, 72°C/160s; then 72°C/10min) the PCR products were analysed by gel electrophoresis to determine the product size. Lysogens containing λJG16C1 expressing *ntr* gave a 1.1kb product whereas λJG3J1 lysogens gave a 0.4kb product. All the colonies gave one or the other of these two bands but not both, and the negative control confirmed the absence of contamination in the PCR.

### Results:

The data are shown in the attached Table 1 and Figures.

The samples from the culture exposed to CB 1954 showed an increase in colony number from 3 to ~360 over the timecourse, corresponding to an increase in the concentration of total bacteriophages released into the medium which increased from 6 x 10⁵ per ml at early times, to 7.2 x 10⁷ per ml at 155 minutes of the timecourse (see Table 1). In the parallel culture not treated with CB1954, the maximum concentration of bacteriophage released was 8 x 10⁶ per ml, at 90 minutes.

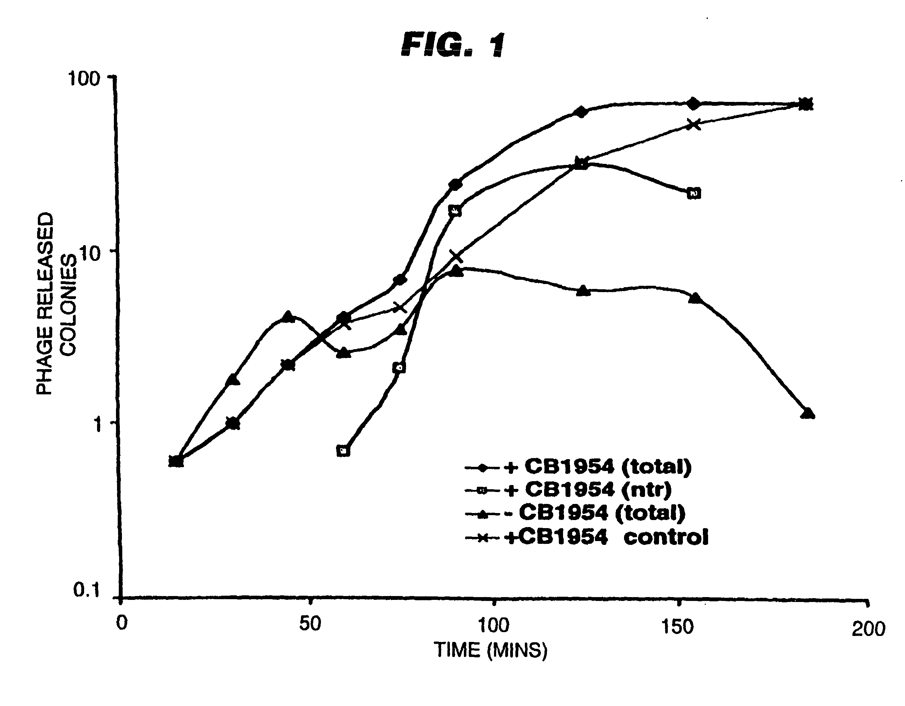

PCR analysis of randomly selected colonies at 90 and 125 minutes of the no-prodrug timecourse, indicated that 1/10 and 0/10 of these contained the λJG16C1 bacteriophage, and the remaining 19/20 contained the λJG3J1 bacteriophage. In contrast, from the culture exposed to CB1954, a far greater proportion of the λJG16C1 bacteriophage (containing the nitroreductase gene) was recovered overall. At 60 minutes, when similar numbers of bacteriophage were recovered ±CB1954, only 1 of 11 colonies screened contained the λG16C1 bacteriophage. However the proportion of λJG16C1 increased rapidly to a peak of 60% (18/30) at 90 minutes, declining thereafter. The peak concentration of the λJG16C1 was reached at 125 minutes (3 x 10⁷ per ml) [see Figure 1].

### Discussion

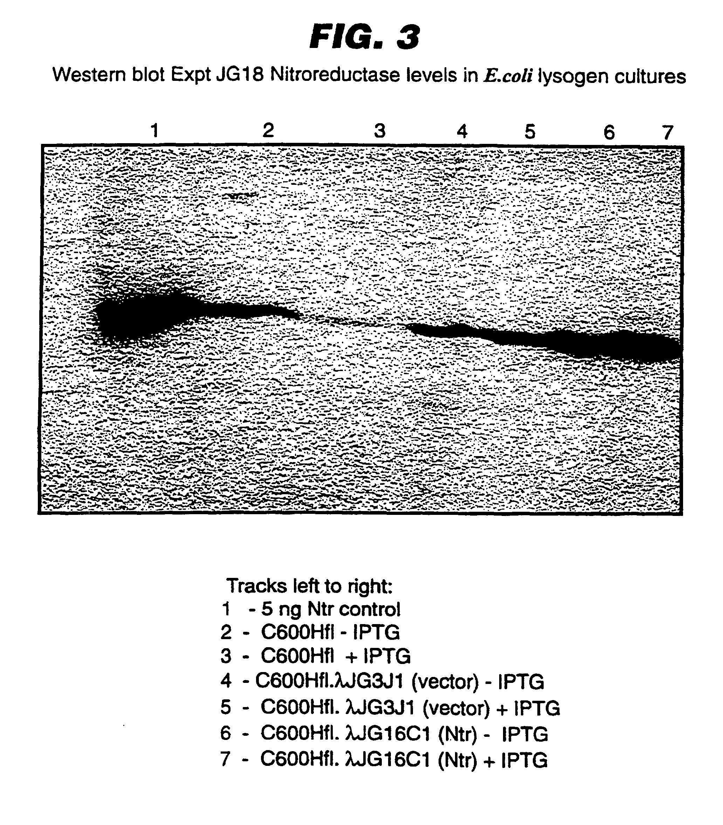

The C600*Hfl* strain of *E*. *coli* contains an endogenous nitroreductase gene. Thus, all the bacteria used in this experiment contain some nitroreductase. This can be visualised as a faint protein band on an SDS-polyacrylamide gel analysis of total bacterial protein and stained with Coomassie blue, and also by blotting such a gel and visualising the nitroreductase selectively, by use of a suitable antibody (data not shown). This has been done for lysogens containing either λJG3J1 or λG16C1, by collecting samples as a timecourse following induction with IPTG. It could thus be estimated that the λJG16C1 lysogen contained around 5-10-fold more nitroreductase when fully induced, than either control cells, or the λJG3J1 lysogen (see Figure 3). The presence of the endogenous nitroreductase in the λJG3J1 lysogen would be expected to lead to some activation of CB 1954; however the 5-10-fold greater level of the enzyme present in the λJG16C1 lysogen would be expected to lead to a faster rate of CB 1954 activation.

The greater number of total bacteriophage released from 60 minutes onwards in the CB1954-treated culture, and the preferential recovery of λJG16C1, provides a strong confirmation that exposure to CB1954 is indeed able to trigger the inactivation of the *c*I repressor leading to lytic bacteriophage growth; and that this is as a consequence of its activation by nitroreductase.

The background level of bacteriophage released into the medium in the culture without CB1954 is expected, as it is known that there can be spontaneous lytic growth from lysogens, without obvious external stimuli. For the first ~60 minutes of exposure to CB1954, there was no increase in the level of bacteriophage above background. Three factors will contribute to this delay; firstly, the amount of CB1954 which is activated and the resulting DNA adducts in the cell will increase with duration of exposure. Second, following activation of the RecA protease, it takes a finite time for this to deplete the level of *c*I sufficiently to trigger lytic bacteriophage growth (perhaps 30 minutes, as reported for induction by ultraviolet light). Third, the duration of the lytic cycle is approximately one hour. The clearly demonstrated preferential recovery of λJG16C1 at times from 75 to 125 minutes can therefore be explained by the faster activation of CB1954 in these cells, leading to earlier triggering of lysis in the cells harbouring λJG16C1 than in those harbouring λJG3J1.

Note that a "bystander effect" has been described in eukaryotic cells, whereby activated prodrug produced in one cell can diffuse and exert an effect upon neighbouring cells that may lack the activating enzyme. It is expected that a similar effect could operate in bacteria. In order to minimise this effect, the experiment was performed at a fairly low initial cell density. It is possible that at later times, a bystander effect has contributed to the induction of lysis in cells with the λJG3J1 prophage. However this experiment has clearly demonstrated that any bystander effect which may be present does not prevent the performance of the selection as described.

This experiment has utilised a vector containing a wild-type nitroreductase gene, in comparison with one lacking a nitroreductase gene, to model the possible distinction that may exist in a library between more- and less-functional nitroreductase genes. In this preliminary experiment, both lysogens contained a background level of nitroreductase contributed by the endogenous gene. Based on the estimate of relative levels of the nitroreductase protein present in two lysogens in the presence of IPTG. we can deduce that the ability of the λJG16C1 lysogen to activate CB1954 would be about 5 -10 fold greater than that of the λJG3J1 lysogen. At the time of mixing. the λJG16C1 formed 16% of the mixture. whereas at the 90 minute time point. 60% of the recovered bacteriophage were λJG16C1, representing a ~4-fold enrichment.

It is predicted that there will be several ways in which this degree of enrichment can be increased.
1) Perform multiple rounds of selection, using the selected bacteriophage from the first round as the input to the second round. It would be expected that a similar degree of enrichment would be obtained, resulting in an enrichment of about 16-fold overall of the λJG16C1 bacteriophage after 2 rounds, 64-fold after 3 rounds, etc.
2) To the extent that the λJG3J1 lysogens were being induced by the endogenous nitroreductase present within those cells rather than a "bystander effect'', it would be possible to decrease the amount of XJG3J1 recovered by using a strain of bacteria in which the endogenous nitroreductase gene had been deleted or inactivated. For example, one could use the *E*. *coli* strain UT5600 (available from the *E*. *coli* Genetic Stock Center, at Yale). This strain does not carry the *Hfl* mutation, and so most of the bacteria initially infected with the bacteriophage would be lysed by the bacteriophage, with release of the progeny bacteriophage. However a proportion of the cells would be lysogenised, and these could be selected either by their survival once the remaining cells had succumbed to lysis, or they could be selected based on the acquisition of kanamycin resistance, or both. Alternatively, it would be possible to inactivate the nitroreductase gene within the C600*Hfl* strain, by a variety of alternative methods.
3) As mentioned earlier, it was not clear at the outset whether lysogens with the imm²¹ *c*I^{ts} would be inducible by DNA damaging agents in the cell. This experiment has shown that it does function adequately in this regard, however it remains possible that an alternative immunity region, such as that of the wild type lambda bacteriophage imm^{λ}*c*I⁺, may allow a greater discrimination.
4) The lytic cycle of lambda takes about one hour to complete, and cleavage of the cI repressor following UV light (and so, perhaps, also in response to the DNA lesions cause by activated CB1954) takes about 30 minutes; thus in this experiment it would appear that activating levels of DNA lesions must have been produced within a few minutes of exposure to CB1954, in the λJG16C1 lysogen, and (5-10-fold slower,) within 30 minutes to 1 hour in the λJG3J1 lysogen. A greater temporal separation between the release of the two types of bacteriophages would be expected if a lower concentration of CB1954 were used, so that the time to induction of the two lysogens would be increased proportionately, giving e.g. a 30 minute delay to the λJG16C1 lysogen and a 2.5-5 hour delay to induction of the λJG3J1 lysogen. The duration of the lytic cycle would be unaffected, and thus there would be less overlap between the release of the two types of bacteriophage.
5) Although it appears unnecessary to invoke significant diffusion of activated prodrug between cells as contributing the induction, it would be possible to further reduce any possible contribution of this by a number of approaches: performing the experiment at a lower cell density; inclusion of substances in the medium to reduce spread of activated prodrug (e.g. serum proteins); or by performing the induction on a solid or semi-solid substrate, e.g. agar plates, such that any activated prodrug released by a cell would only diffuse a short distance, rather than being spread throughout the culture as in a shaking liquid culture.

The principles established by this experiment indicate that the selection method and vector will also be effective when applied to a library of nitroreductase variants. In doing so, this experiment has shown that it would be desirable to perform the induction with lower concentrations of CB1954 (e.g. 100µM or lower), and to collect the released bacteriophages at intervals. The number of bacteriophage particles released would be monitored and compared with parallel cultures lacking CB1954; this would inform the decision which time-point sample to choose for further rounds of selection. One would perform several rounds of selection (e.g. 3-5) in order to reduce the level of "background'' bacteriophages present; and then recover the inserts from the bacteriophages, e.g. by PCR, to enter them in further rounds of PCR-shuffling.

### References

1. **Knox, R. J.** (Personal communication.
2. **Hendrix, R. W., J. W. Roberts, F. W. Stahl, and R. A. Weisberg.** (1983) Lambda II.
3. **Murray, N. E.** (1983) bacteriophage lambda and molecular cloning, p. 395-431. *In* Hendrix (ed.), Lambda II. Cold Spring Harbor Laboratory Press,
4. **Prentki, P., and H. M. Krisch**. (1984) In vitro insertional mutagenesis with a selectable DNA fragment. Gene **29**:303-313.
5. **Workman, P., R. A. S. White, and K. Talbot**. (1986) CB1954 revisited. I. Disposition kinetics and metabolism. Cancer Chemother. Pharmacol. **16**:1-8.
6. **Sambrook, J., E. F. Fritsch, and T. Maniatis**. (1989) Molecular Cloning: A Laboratory Manual 2nd edition.
7. **Boland, M. P., R. J. Knox, and J. J. Roberts.** (1991) The differences in kinetics of rat and human DT diaphorase result in a differential sensitivity of derived cell lines to CB1954 (5-(aziridin-1-yl)-2,4-dinitrobenzamide). Biochem. Pharmacol. **41**:867-875.
8. **Huber, B. E., C. A. Richards, and T. A. Krenitsky.** (1991) Retroviral-mediated gene therapy for the treatment of hepatocellular carcinoma: an innovative approach for cancer therapy. Proc. Natl. Acad. Sci. USA **88**:8039-8043.
9. **Anlezark, G. M., R. G. Melton, R. F. Sherwood, B. Coles, F. Friedlos, and R. J. Knox.** (1992) The bioactivation of 5-(aziridin-1-yl)-2,4-dinitrobenzamide (CB 1954)―I. Purification and properties of a nitroreductase enzyme from *Escherichia coli-* a potential enzyme for antibody-directed enzyme prodrug therapy (ADEPT). Biochem. Pharmacol. **44**:2289-2295.
10. **Harris, J. D., A. A. Gutierrez, H. C. Hurst, K. Sikora, and N. R. Lemoine.** (1994) Gene therapy for cancer using tumour-specific prodrug activation. Gene Therapy **1**:170-175.
11. **Hubcr, B. E., E. A. Austin, C. A. Richards, S. T. Davis, and S. S. Good.** (1994) Metabolism of 5-fluorocytosine to 5-fluorouracil in human colorectal tumour-cells transduced with the cytosine deaminase gene ― significant anti-tumour effects when only a small percentage of tumour-cells express cytosine deaminase. Proc. Natl. Acad. Sci. USA **91**:8302-8306.
12. **Stemmer, W. P. C**. (1994) Rapid evolution of a protein *in vitro* by DNA shuffling. Nature **370**:389-391.
13. **Anlezark, G. M., R. G. Melton, R. F. Sherwood, W. R. Wilson, W. A. Denny, B. D. Palmer, R. J. Knox, F. Friedlos, and A. Williams.** (1995) Bioactivation of dinitrobenzamide mustards by an *E. coli* B nitroreductase. Biochem. Pharmacol. **50**:609-618.
14. **Black, M. E., T. G. Newcomb, H. M. P. Wilson, and L. A. Loeb.** (1996) Creation of drug-specific herpes simplex virus type 1 thymidine kinase mutants for gene therapy. Proc. Natl. Acad. Sci. USA **93**:3525-3529.
15. **Searle, P. F., N. K. Green, L. S. Young, D. J. Kerr, and J. P. Neoptolemos.** (1996) Gene transfer therapy and pancreatic cancer, p. 194-213. *In* J. P. Neoptolemos and N. Lemoine (ed.), Pancreatic cancer: Molecular and clinical advances. Blackwell Science, Oxford.
16. **Chen, S., R. Knox, K. Wu, P. S.-K. Deng, D. Zhou, M. A. Bianchet, and L. M. Amzel.** (1997) Molecular basis of the catalytic differences among DT-diaphorase of human, rat and mouse. J. Biol. Chem. **272**:1437-1439.
17. **Friedlos, F., W. A. Denny, B. D. Palmer, and C. J. Springer.** (1997) Mustard prodrugs for activation by *Escherichia coli* nitroreductase in gene-directed enzyme prodrug therapy. Journal of Medicinal Chemistry **40**:1270-1275.
**18. Green, N. K., D. J. Youngs, J. P. Neoptolemos, F. Friedlos, R. J. Knox, C. J. Springer, G. M. Anlezark, N. P. Michael, R. G. Melton, M. J. Ford, L. S. Young, D. J. Kerr, and P. F. Searle.** (1997) Sensitization of colorectal and pancreatic cancer cell lines to the prodrug 5-(aziridin-1-yl)-2,4-dinitrobenzamide (CB1954) by retroviral transduction and expression of the *E*. *coli* nitroreductase gene. Cancer Gene Therapy **4**:229-238.
19. **Crameri, A., S. A. Raillard, E. Bermudez, and W. P. C. Stemmer.** (1998) DNA shuffling of a family of genes from diverse species accelerates directed evolution. Nature **391**:288-291.
20. **McNeish, I. A., N. K. Green, M. G. Gilligan, M. J. Ford, V. Mautner, L. S. Young, D. J. Kerr, and P. F. Searle.** (1998) Virus directed enzyme prodrug therapy for ovarian and pancreatic cancer using retrovirally delivered *E*. *coli* nitroreductase and CB1954. Gene Therapy **5**:1061-1069.

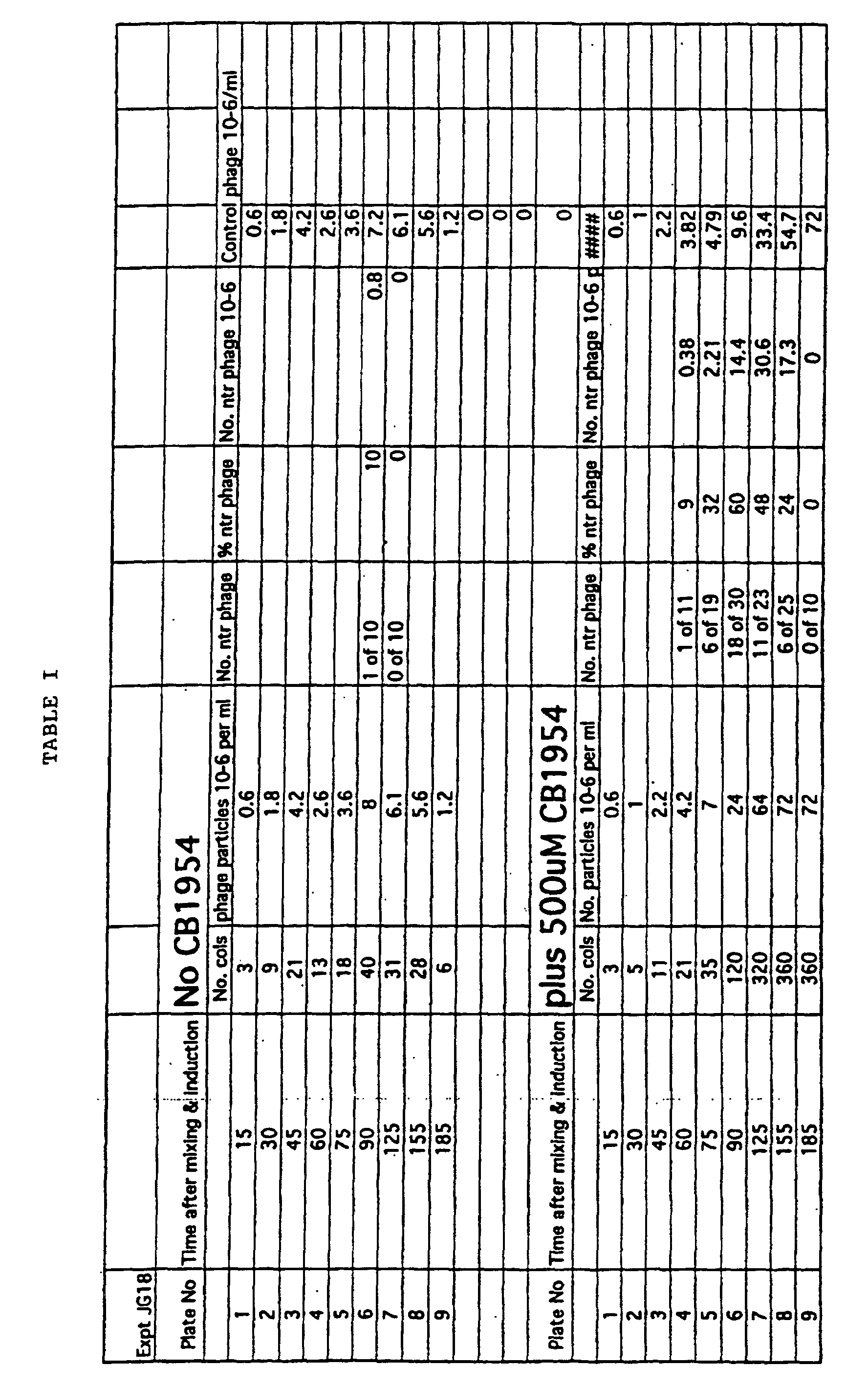

## Claims

1. A method of selection of nucleic acid encoding an enzyme that is capable of converting a prodrug to its active drug form comprising the steps of:
a) contacting a population of bacteria transformed with a bacteriophage library with a prodrug in a medium, wherein;
i) the transformed bacteria are in the lysogenic state, and
ii) when converted to its active drug form, the prodrug causes activation of the proteolytic activity of bacterial RecA and lysis of the bacteria;
b) separating bacteriophage particles released by lysis of the bacteria from said medium, and
c) analysing the genotype of said separated bacteriophage for a nucleic acid encoding the enzyme.

2. A method according to claim 1 wherein said steps are repeated in at least one cycle.

3. A method according to any one of the preceding claims wherein the DNA sequence of said released bacteriophage particles is analysed

4. A method according to any one of the preceding claims wherein said bacteriophage carry a gene encoding antibiotic resistance or other selectable marker.

5. A method according to any one of the preceding claims wherein said enzyme is nitroreductase, flavin reductase, DT-diaphorase, thymidine kinase, cytosine deaminase or a purine nucleoside phosphorylase

6. A method according to any one of the preceding claims wherein said prodrug is CB1954, SN 23862, 2-(N,N-bis(2-iodoethyl)amino]-3,5-dinitrobenzamide, 5-fluorocytosine, acyclovir, ganciclovir, or 6-methyl-9-(2-deoxy-β-D-erythro-pentofuranosyl) purine.

7. A method according to any of the preceding claims wherein said bacteriophage is the bacteriophage lambda or a lambda derivative.

8. A method according to any one of claims 1-7 wherein said bacteriophage library comprises genes encoding variants of a single enzyme.

9. A method according to claim 8 wherein said variants comprise amino acid deletions, insertions and substitutions from the wild type enzyme sequence.

10. A method according to claim 8 or 9 wherein said variants are generated by DNA shuffling, random mutagenesis, or PCR shuffling.

11. A method according to any one of the preceding claims wherein said activity of said bacterial RecA protein is caused by the generation of single-stranded DNA in the bacterium.

12. A method according to claim 11 wherein said single-stranded DNA is generated as a consequence of the enzymatic conversion of prodrug to its active drug form.

13. A method according to claim 12 wherein said single-stranded DNA arises as a result of a break in one or both strands of the DNA, a cytotoxic lesion, a DNA crosslink or a monovalent DNA adduct, or by inhibition of the progress of DNA replication by any other means.

14. A method according to any one of the preceding claims wherein said enzyme comprises nitroreductase and said prodrug comprises CB1954.

15. A method according to any one of the preceding claims wherein said bacteriophage vector is λJG3JI.

16. A method according to any one of the preceding claims wherein said bacterium is of the *E.coli* strain C600*Hfl*.

## Patentansprüche

1. Verfahren zur Auswahl von Nukleinsäure, die ein Enzym kodiert, das in der Lage ist, eine Prodrug in ihre Wirkstoff-Form umzuwandeln, wobei das Verfahren folgende Schritte umfaßt:
a) Kontaktieren einer Bakterienpopulation, die mit einer Bakteriophagen-Bank transformiert wurde, mit einem Prodrug in einem Medium, wobei
i) die transformierten Bakterien in lysogenem Zustand vorliegen, und
ii) das Prodrug, wenn es in seine Wirkstoff-Form umgewandelt wurde, eine Aktivierung der proteolytischen Aktivität von bakterieller RecA und eine Lyse der Bakterien verursacht;
b) Abtrennen von Bakteriophagen-Teilchen, die durch Lyse der Bakterien aus dem Medium freigesetzt werden, und
c) Analysieren des Genotyps der abgetrennten Bakteriophagen hinsichtlich einer Nukleinsäure, welche das Enzym kodiert.

2. Verfahren nach Anspruch 1, bei dem die Schritte in mindestens einem Zyklus wiederholt werden.

3. Verfahren nach einem der vorhergehenden Ansprüche, bei dem die DNA-Sequenz der freigesetzten Bakteriophagenteilchen analysiert wird.

4. Verfahren nach einem der vorhergehenden Ansprüche, bei dem die Bakteriophagen ein Gen, das für antibiotische Resistenz kodiert, oder einen anderen selektierbaren Marker tragen.

5. Verfahren nach einem der vorhergehenden Ansprüche, bei dem das Enzym Nitroreduktase, Flavinreduktase, DT-Diaphorase, Thymidin-Kinase, Cytosin-Deaminase oder eine Purinnukleosidphosphorylase ist.

6. Verfahren nach einem der vorhergehenden Ansprüche, wobei die Prodrug CB1954, SN 23862, 2-[N,N-Bis(2-iodethyl)amino]-3,5-dinitrobenzamid, 5-Fluorcytosin, Acyclovir, Ganciclovir oder 6-Methyl-9-(2-desoxy-β-D-erythro-pentofuranosyl)purin ist.

7. Verfahren nach einem der vorhergehenden Ansprüche, bei dem der Bakteriophage der Bakteriophage Lambda oder ein Lambda-Derivat ist.

8. Verfahren nach einem der Ansprüche 1 bis 7, bei dem die Bakteriophagen-Bank Gene umfaßt die Varianten eines einzelnen Enzyms kodieren.

9. Verfahren nach Anspruch 8, bei dem die Varianten Aminosäure-Deletionen, -Insertionen und -Substitutionen der Wildtyp-Enzymsequenz umfassen.

10. Verfahren nach Anspruch 8 oder 9, bei dem die Varianten durch DNA-Shuffling, Zufallsmutagenese oder PCR-Shufiling erzeugt werden.

11. Verfahren nach einem der vorhergehenden Ansprüche, bei dem die Aktivität des bakteriellen RecA-Proteins durch die Erzeugung einer einsträngigen DNA in dem Bakterium verursacht wird.

12. Verfahren nach Anspruch 11, bei dem die einsträngige DNA infolge der enzymatischen Umwandlung der Prodrug in ihre Wirkstoff-Form erzeugt wird.

13. Verfahren nach Anspruch 12, bei dem die einsträngige DNA infolge eines Bruchs in einem Strang oder in beiden Strängen der DNA, durch eine cytotoxische Läsion, eine DNA-Vernetzung oder ein monovalentes DNA-Addukt oder durch Inhibierung des Fortschreitens der DNA-Replikation mit anderen Mitteln entsteht.

14. Verfahren nach einem der vorhergehenden Ansprüche, bei dem das Enzym Nitroreduktase umfaßt und das Prodrug CB1954 umfaßt.

15. Verfahren nach einem der vorhergehenden Ansprüche, bei dem der Bakteriophagenvektor λJG3JI ist.

16. Verfahren nach einem der vorhergehenden Ansprüche, bei dem das Bakterium eines vom *E. coli*-Stamm C600 *Hfl* ist.

## Revendications

1. Méthode de sélection d'un acide nucléique encodant une enzyme qui est apte à transformer un promédicament dans sa forme médicamenteuse active comprenant les étapes de :
a) mise en contact d'une population bactérienne transformée avec une chimiothèque bactériophage avec un promédicament dans un milieu, où :
i) les bactéries transformées sont dans l'état lysogène, et
ii) quand il est transformé en sa forme médicamenteuse active, le promédicament entraîne l'activation de l'activité protéolytique du RecA bactérien et la lyse de la bactérie.
b) séparation des particules bactériophages libérées par lyse des bactéries à partir dudit milieu, et
c) analyse du génotype dudit bactériophage séparé pour un acide nucléique encodant l'enzyme.

2. Méthode selon la revendication 1, **caractérisée en ce que** lesdites étapes sont répétées selon au moins un cycle.

3. Méthode selon l'une des revendications précédentes, **caractérisée en ce que** ladite séquence ADN desdites particules bactériophages libérées est analysée.

4. Méthode selon l'une des revendications précédentes, **caractérisée en ce que** lesdits bactériophages portent un gène encodant une résistance antibiotique ou d'autres marqueurs choisissables.

5. Méthode selon l'une des revendications précédentes, **caractérisée en ce que** ladite enzyme est nitroreductase, reductase de flavine, DT-diaphorase, kinase de thymidine, déaminase de cytosine ou un phosphorylase nucléoside purine.

6. Méthode selon l'une des revendications précédentes, **caractérisée en ce que** ledit promédicament est CB1954, SN 23862, 2-[N,N-bis(2-iodoéthyle)amino]-3,5-dinitrobenzamide, 5-fluorocytosine, acyclovir, ganciclovir, ou 6-méthyle-9-(2-déoxy-β-D-érythropentofuranosyl) purine.

7. Méthode selon l'une des revendications précédentes, **caractérisée en ce que** ledit bactériophage est le bactériophage lambda ou un dérivé lambda.

8. Méthode selon l'une des revendications 1 à 7, **caractérisée en ce que** ladite chimiothèque bactériophage comprend des gènes encodant des variantes d'un enzyme simple.

9. Méthode selon la revendication 8, **caractérisée en ce que** lesdites variantes comprennent des délétions, insertions et substitutions d'acides aminés à partir de la séquence d'enzymes de type sauvage.

10. Méthode selon la revendication 8 ou 9, **caractérisée en ce que** lesdites variantes sont générées par un réarrangement d'ADN, une mutagénèse aléatoire, ou un réarrangement PCR.

11. Méthode selon l'une des revendications précédentes, **caractérisée en ce que** ladite activité de ladite protéine RecA bactérienne est causée par la génération d'un ADN à simple brin dans la bactérie.

12. Méthode selon la revendication 11, **caractérisée en ce que** ledit ADN à simple brin est généré comme la conséquence de la transformation enzymatique du promédicament en sa forme médicamenteuse active.

13. Méthode selon la revendication 12, **caractérisée en ce que** ledit ADN à simple brin apparaît comme un résultat d'une cassure dans un ou les deux brins de l'ADN, une liaison cytotoxique, une réticulation d'ADN ou un ajout d'ADN monovalent, ou par inhibition du processus de réplication d'ADN par tout autre moyen.

14. Méthode selon l'une des revendications précédentes, **caractérisée en ce que** ledit enzyme comprend de la nitroréductase et ledit promédicament comprend CB1954.

15. Méthode selon l'une des revendications précédentes, **caractérisée en ce que** ledit vecteur bactériophage est λJG3JI.

16. Méthode selon l'une des revendications précédentes, **caractérisée en ce que** ladite bactérie est de la C600Hfl souche E.coli.
